# EUROPEAN PATENT APPLICATION

(11) **EP 1 616 585 A1**
(43) Date of publication of application: **18.01.2006**
(21) Application number: 05014370.0
(22) Date of filing: 01.07.2005
(51) Int. Cl.: A61L 31/12, A61L 31/16, A61F 2/06

(54) **Device for the treatment of aneurysms**

(30) Priority: 02.07.2004 US 585283 P; 03.08.2004 US 910009
(71) Applicant: Medtronic Vascular, Inc., Santa Rosa, CA 95403 (US)
(72) Inventor: Tijsma, Edze Jan, 6224 LD Maastricht (NL); Gillissen, Mirian, 6271 AJ Gulpen (NL); Kwitkin, Brian, 6221 JC Maastricht (NL)
(74) Representative: Zimmermann, Gerd Heinrich

(57) **Abstract**

A method and device for the treatment of an aneurysm are provided. Different therapeutic agents are delivered to the aneurysmal site by a delivery vehicle in a localized, time-release regimen, to treat and reduce the severity of the aneurysm. The delivery vehicle is provided in conjunction with the placement of an excluding device, such as a stent graft.

## Description

### FIELD OF THE INVENTION

The field of the invention is the treatment of vascular abnormalities. More specifically, the field of the invention is the treatment of vascular abnormalities using therapeutic agents delivered to the aneurysmal site by a delivery vehicle.

### BACKGROUND OF THE INVENTION

### Description of the Related Art

Aneurysm, the enlargement of a blood vessel at a specific location--at times to the point where rupture of the blood vessel is imminent--has been treated in the past by surgical intervention techniques, where the affected portion of the blood vessel is removed or bypassed so that the vessel lumen is replaced by a synthetic graft. This treatment regimen is highly invasive, typically requiring a multiple day post-operative hospital stay, as well as several months of recuperative time.

Prophylactic methods for preventing the formation of aneurysm tend to rely on reducing blood pressure in an effort to reduce mechanical stress on the blood vessels. These methods involve drugs that can have undesirable side effects, e.g., kidney or liver damage, especially over long-term use.

To address the limitations imposed by surgical intervention or drug therapy, a technique has been developed by which the aneurysmal blood vessel site is treated by placing what is known in the art as a stent graft within the blood vessel in a position where the tubular body of the stent graft spans the interior of the weakened area of the blood vessel wall. The stent graft, properly positioned, allows blood to flow through the hollow tubular interior of the stent graft preventing blood, under systemic pressure, from reaching the weakened blood vessel wall at the aneurysmal site. However, there is still an opportunity for blood to reach the weakened wall location, such as through leakage of blood between the seal formed between the end of the stent graft and the vessel wall, by diffusion of blood through the graft material, or through the supply of blood into the aneurysmal sac from adjacent blood vessels. In each case, there is a renewed risk that the blood vessel may rupture. Furthermore, there remains a risk of additional deterioration of the blood vessel wall at the aneurysmal location even in the absence of blood leakage.

Typically, surgical intervention for aneurysm repair is not indicated until the blood vessel diameter at the aneurysmal site expands to 200 to 300% of its normal diameter. Below this threshold, the normal course of treatment has been to monitor the site, and if the diameter of the blood vessel wall at the aneurysmal site continues to expand beyond an undesirable threshold diameter, intervene surgically. Through numerous studies performed during the past decade, which were summarized by Thompson et al. in Curr. Probl. Surg., 39, 93 (2002), the basic knowledge of aneurysm disease has increased significantly. Despite this increase no therapeutic approaches have proven to reduce the rate of aneurysms in patients. Given the fact that several pharmacological strategies, e.g., cyclooxygenase-2 (COX-2) inhibitors, tissue inhibitors of matrix metalloproteinases (MMP), and antibiotics, can reduce the severity and/or progression of an aneurysm in animal models, these approaches may reduce the likelihood of the need for surgical intervention to repair the aneurysm.

Based on the current knowledge of the causes of aneurysms, MMP inhibition has been proposed as a potential therapeutic approach to treat abdominal aortic aneurysm (AAA). Typical treatment requires the use of systemic MMP inhibitors, either orally, intra-muscularly or intra-venously introduced, in a dosage sufficient to ensure that the quantity of agent reaching the aneurysm is sufficient to affect level of the elastin-attacking protein. For example, doxycycline could inhibit AAA growth in humans (see J. Vasc. Surg., 31, 325 (2000)); however, the side effects of doxycycline, including the likelihood of the patient developing doxycycline resistant bacteria as a result of long term doxycycline treatment, dictate that doxycycline treatment may be only a short term solution to the problem of aneurysm growth. Other approaches to treat AAA are the use of anti-inflammatory agents, including COX-2 inhibitors (see Circulation, 100, 48 (1999)), angiotensin-converting enzyme (ACE) inhibitors (see J. Vasc. Surg., 33, 1057 (2001)), nitric oxide synthase (NOS) inhibitors (see Ann. Vasc. Surg., 16, 65 (2002)), and antioxidants.

In view of the above considerations, it is clear that the present methods to treat AAA have limitations. Moreover, there is no practical pharmacological method available to prevent the aneurysm formation, without having to worry about the side effects of the drugs used. US Pat. No. 5,834,449 to Thompson employs the use of a tetracycline compound having no antimicrobial activity; proposing use of a medical apparatus capable of intravascular delivery; however, no details on the device are disclosed.

Thus, there exists a need, post-placement of a stent graft, to treat an aneurysmal site with therapeutic agents, so as to reduce the severity and/or the progression of the aneurysm and the risk of aneurysm rupture, tear or other failure. Therefore, there exists a need in the art to develop additional treatment regimens, particularly non-invasive or minimally invasive localized techniques, which may be used to reduce the severity of aneurysm. Therefore, it is an object of the present invention to provide an improved device for treatment of aneurismal tissue.

### SUMMARY OF THE INVENTION

This object is solved by a device according to claims 1 and 16. Further aspects, advantages, features and details of the present invention are apparent from the dependent claims, the specification, and the accompanying drawings. Embodiments according to the present invention provide localized application of therapeutic agents useful to reduce the severity and the progression of an aneurysm at an aneurysmal site. One embodiment includes the administration of one or more therapeutic agents selected from a class of therapeutic agents which have been shown to block or inhibit the formation of biochemical compounds found in aneurysmal sites but not found--or found in much smaller quantities--in healthy blood vessels by using a specialized delivery vehicle. The therapeutic agents may include matrix metalloproteinase (MMP) inhibitors, antibiotics, cyclooxygenase-2 (COX-2) inhibitors, angiotensin-converting enzyme (ACE) inhibitors, glucocorticoids, beta blockers, nitric oxide synthase (NOS) inhibitors, antioxidants, non-steroidal anti-inflammatory drugs (NSAIDs) and cellular adhesion molecules (CAMs), and combinations of these. The agents preferably are localized to (adjacent) the aneurysmal site by the placement of a delivery vehicle that is comprised of, or within which is provided, a carrier containing and capable of dosing over time the therapeutic agents. The materials to be used for such a carrier can be synthetic polymers, natural polymers, inorganics and combinations of these.

The physical form of the drug-loaded carrier can be a sheet, coating, slab, gel, capsule, microparticles, nanoparticles and combinations of these. In one embodiment the carrier is placed in a pouch that is attached to or wrapped around the outer--i.e. blood vessel wall side--of a stent graft passing through an aneurysmal blood vessel. The stent graft isolates the aneurysmal region of the blood vessel from blood flow and provides a structure on which to attach the delivery device so that the agent may be delivered directly to the aneurysmal blood vessel site. The delivery device is positioned on or wrapped around a stent, stent graft, or other intervention device spanning the aneurysmal site through the interior of a blood vessel to release therapeutic agents into the space between the intervention device and the wall of the aneurysmal blood vessel. In another embodiment the stent graft comprises two layers of fabric, and the drug-loaded carrier is placed inside such a double-walled stent graft.

The delivery vehicle should be permeable to water with and without dissolved therapeutic agents and it should be biocompatible. In a preferred embodiment the carrier is biodegradable, where the release of the therapeutic agents is governed by a combination of diffusion and degradation of the carrier, thus releasing the therapeutic agent over time. The delivery vehicle is positioned at the aneurysmal site, such as by inclusion on or in an exclusion device such as a stent graft.

### BRIEF DESCRIPTION OF THE DRAWINGS

So that the above-recited features according to the present invention can be understood in detail, a more particular description may be had by reference to embodiments, some of which are illustrated in the appended drawings.

Figure 1 is a sectional view of an descending aorta with a stent graft placed therein;

Figure 2A is an enlarged view of a portion of the stent graft of Figure 1 including one embodiment of a delivery device;

Figure 2B is an enlarged view of a portion of a stent graft positioned in a descending aorta where the aneurysm is less advanced than as seen in Figure 2A;

Figure 3a is an enlarged side view of the delivery device in Figure 2. Figure 3b is a top view of the delivery device shown in Figure 2.

### DETAILED DESCRIPTION

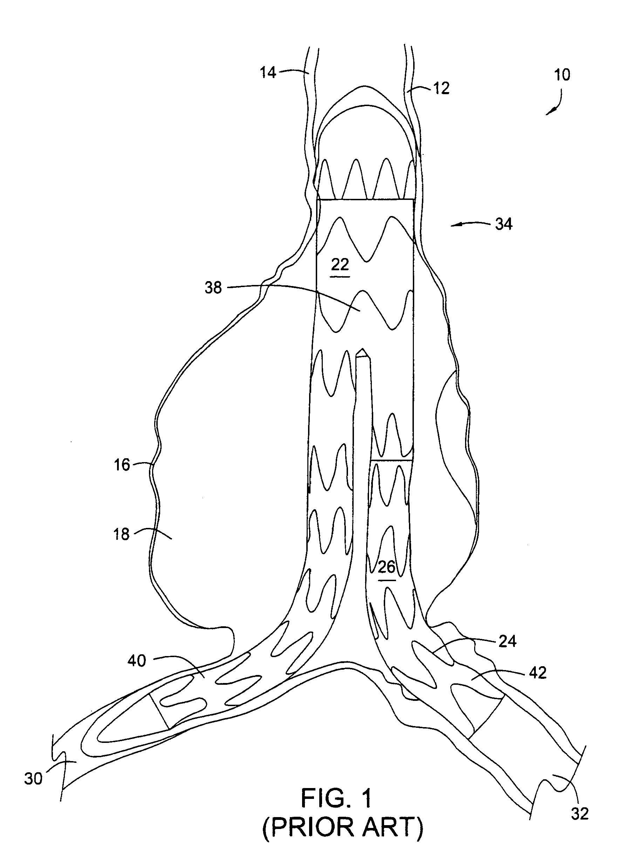

An embodiment according to the present invention provides a delivery device or vehicle to deliver locally therapeutic agents at the site of an aneurysm, preferably in a time-release formulation. Referring first to Figure 1, there is shown, in section, an aneurysmal blood vessel, in this instance a descending aorta 10. Aorta 10 includes a wall 12, having a healthy wall portion 14 and an aneurysmal wall portion 16, wherein the aneurysmal wall portion 16 occurs where the aorta has a diameter substantially larger than it does where the healthy wall portion 14 occurs. Aneurysmal portion 16 forms an aneurysmal bulge or sac 18, wherein the elastin in the extra-cellular matrix of the aortic vessel wall 12 is degraded, preventing the aortic wall 12 at the aneurysmal portion 16 from holding the aorta at its healthy diameter against the pressure of blood.

Where the aneurysmal sac 18 has progressed to a diameter on the order of more than twice to three times the diameter of the healthy aortic wall 14, intervention to prevent rupture of the aneurysm is dictated. Surgical intervention can include highly invasive procedures, where the section of the aorta undergoing the aneurysmic event is opened up or removed completely, and a synthetic graft is sewn in place between healthy sections of the aorta or the severed ends of the aorta (not shown). Alternatively intervention may encompass exclusion of the aneurysmal sac 18 by placement of an exclusion device such as a stent graft 22 (a modular bifurcated stent graft being shown here). The stent graft typically includes a stent portion 24, having a supportive yet collapsible construction (here in a grid pattern), to which a graft portion 26 is sewn or attached. The stent portion 24 provides a tubular body having a support capability sufficient to hold the graft portion 26 in an open position across the aneurysmal sac 18, such that the opposed ends are received and sealed against healthy portions 14 of the of the aorta. The graft portion 26 blocks the passage of blood to the aneurysmal sac 18, and provides a conduit for blood flow past the aneurysmal sac 18.

Although the stent graft 22 provides an exclusionary environment through which blood may flow past the aneurysmal sac 18, there remains a need to treat the aneurysmal sac 18. In particular, it is known that fresh blood may leak into the aneurysmal sac 18 region, despite the presence of stent graft 22, leading to further breakdown in the extra-cellular matrix and the aneurysmal vessel. If this occurs, the excluded aneurysmal vessel may rupture leading to patient mortality. Therefore, there remains a need to treat the aneurysmal sac 18 further despite the presence of the excluding device or as an alternative to using an excluding device.

In many embodiments, one or more therapeutic agents described herein, *infra,* are provided in a delivery vehicle included with an excluding device or intravascular repair vehicle, for example, the stent graft 22 shown in Figure 1. Referring back to Figure 1, the placement of the stent graft 22 in the aorta 10 is a technique well known to those skilled in the art, and essentially includes the opening of a blood vessel in the leg, and the insertion of the stent graft 22 contained in a catheter into the vessel, guiding the catheter through the vessel, and deploying the stent graft 22 in a position spanning the aneurysmal sac 18.

Although the bifurcated stent graft 22 is shown in its fully assembled and positioned state, it is to be understood that the bifurcated stent graft 22 typically is considered to have at least three portions, a trunk portion 38, located in the lower portion of the descending aorta 34, and two minor diameter leg portions 40, 42 that fit into the two legs of the iliac arteries 30 and 32. In one embodiment, the bifurcated stent graft 22 is configured such that the trunk and leg portion 38, 40 and 42 each include a graft portion, supported externally by a tubular metal stent portion that each expand to a pre-established diameter when placed in the vasculature.

When assembled in situ, the entire stent graft 22 spans the aneurysmal sac 18 in the aorta 10, to seal the aneurysmal portion of the aorta 10 from blood flowing through the aorta 10. The metal stent 24 includes a plurality of hoop frame members each of which preferably includes a plurality of elements, here, diamond-shaped which surround the circumference of the graft at a particular location where the hoop forms a portion of the stent structure as a whole, e.g., the Medtronic AneuRx® stent graft.

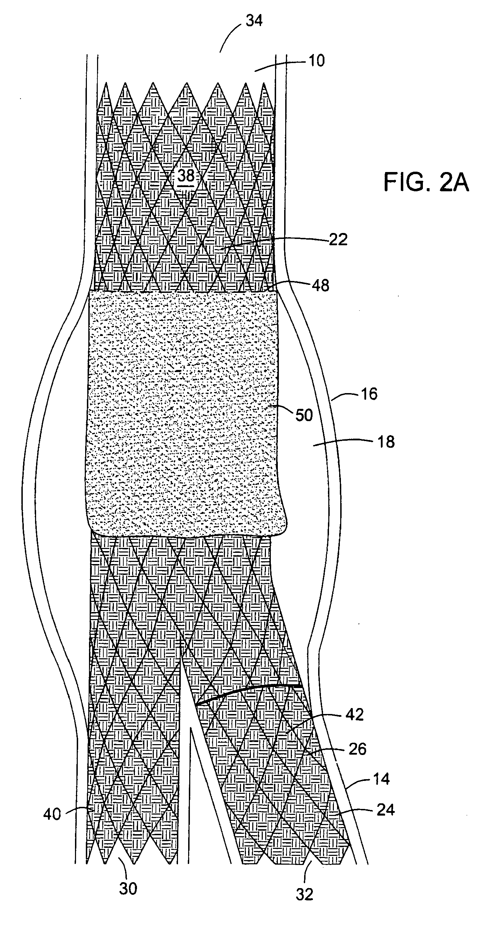

Turning to Figure 2A, there is shown a therapeutic agent delivery device in the form of a delivery vehicle 50, connected to a ring 48 on the outer surface of the stent graft 22. The delivery vehicle 50 is positioned such that upon placement in an aneurysmal blood vessel location such as aneurysmal sac 18 of aorta 10, the delivery vehicle 50 is located between the stent graft 22 and the aneurysmal wall 16 of aorta 10. The delivery vehicle 50 preferably is positioned on the stent graft 22 to ensure a proximal positioning with healthy tissue in the wall 16 of the aorta 10.

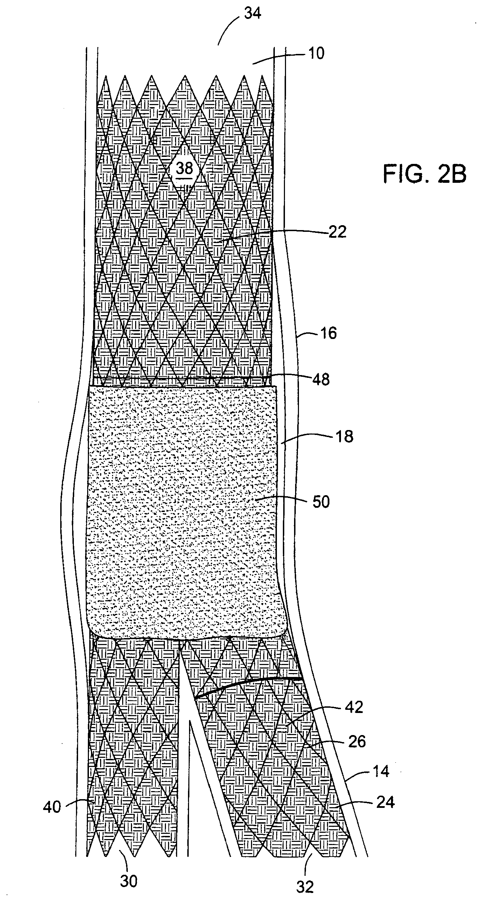

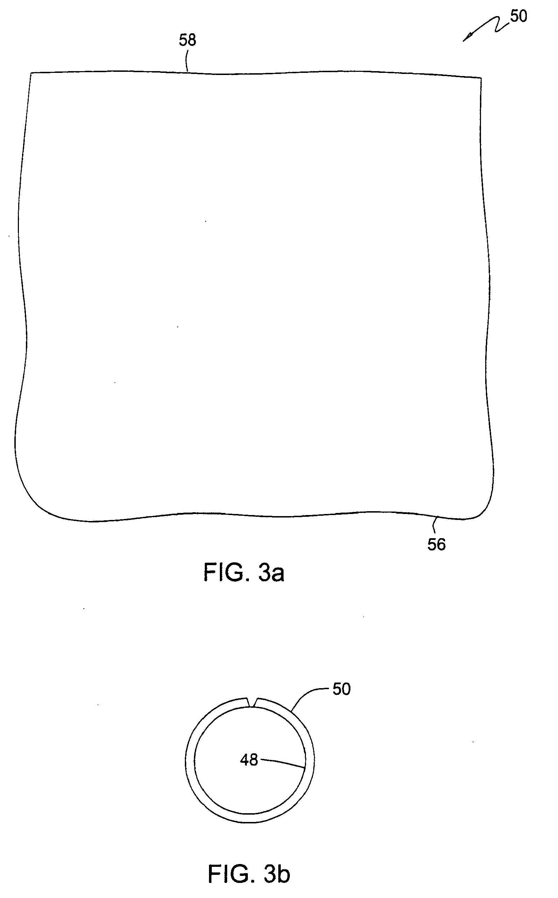

Referring now to Figure 3a, the delivery vehicle in the form of a pouch 50 from Figure 2 is shown. Here, the delivery vehicle 50 is shown as a wide bag or sac that eventually is wrapped around the outer diameter surface of a stent graft, attached at end 58 of delivery vehicle 50. The delivery vehicle may carry a therapeutic agent formulated with a carrier in the form of a sheet, coating, slab, gel, capsule, microparticles, nanoparticles and/or combinations of thereof. To maintain the therapeutic agent within the delivery vehicle 50 yet allow transport of fluids and agent through the delivery vehicle 50, the delivery vehicle is manufactured from a biocompatible material that is permeable to water and dissolved therapeutic agents. The pouch can be manufactured from materials such as polyolefins (polypropylene, polyethylene), polyurethanes, silicones, polyesters (Dacron@) and fluorinated polyolefins (PTFE). Delivery vehicle 50 may be prepared, for example, by folding a sheet of the pouch material in half, and attaching together the opposed sides projecting from the crease occurring at the fold which forms end 56, such as by sewing, laser welding, adhesives or the like to leave an open end. The therapeutic agent formulation is then loaded into the interior of the delivery vehicle 50, and the open end 58 is then sealed by similar means as the means used to close the sides. Figure 3b shows a top view of delivery vehicle 50 attached to ring 48 of the stent graft 22.

The therapeutic agent/carrier formulation comprises a material to ensure the controlled release of the therapeutic agent. The materials to be used for such a formulation--as well as the delivery vehicle itself, in some embodiments--are preferably comprised of a biocompatible polymer, in which the therapeutic agent is present. The dispersion of the therapeutic agent allows the therapeutic reaction to be substantially localized so that overall dosages to the individual can be reduced, and undesirable side effects caused by the action of the agent in other parts of the body are minimized.

Thus, the therapeutic agent formulation comprises a carrier, which according to the present invention can be made of synthetic polymers, natural polymers, inorganics and combinations of these. The polymers may be either biodegradable or non-biodegradable. Typical examples of biodegradable synthetic polymers are listed below:
- Aliphatic polyesters, such as poly(lactic acid), poly(glycolic acid), poly(lactic acid-co-glycolic acid), poly(ε-caprolactone), poly(trimethylene carbonate), polydioxanone and copolymers; poly(hydroxy butyrate) (Biopol®), poly(hydroxy valerate), poly(hydroxy butyrate-co-hydroxy valerate), poly(butylene succinate) (Bionolle®), poly(butylene adipate),
- Polyanhydrides, such as poly(adipic anhydride), and poly(sebacic acid-co-1,3-bis(p-carboxyphenoxy)propane),
- Poly(ortho ester)s,
- Poly(ester amide)s, such as based on 1,4-butanediol, adipic acid, and 1,6-aminohexanoic acid (BAK 1095),
- Poly(ester urethane)s,
- Poly(ester anhydride)s,
- Poly(ester carbonate)s, such as tyrosine-poly(alkylene oxide)-derived poly(ether carbonate)s,
- Polyphosphazenes,
- Polyarylates, such as tyrosine-derived polyarylates,
- Poly(ether ester)s, such as poly(butylene terephthalate)-poly(ethylene glycol) copolymers (PolyActive®), poly(ε-caprolactone)-b-poly(ethylene glycol)) block copolymers, and poly(ethylene oxide)-b-poly(hydroxy butyrate) block copolymers.

Examples of biostable synthetic polymers include:
- Polyolefins, such as polyethylene, polypropylene,
- Polyurethanes,
- Fluorinated polyolefins, such as polytetrafluorethylene (Teflon®),
- Chlorinated polyolefins, such as poly(vinyl chloride),
- Polyamides,
- Acrylate polymers, such as poly(methyl methacrylate) and copolymers (Eudragit®),
- Acrylamide polymers, such as poly(N-isopropylacrylamide),
- Vinyl polymers, such as poly(N-vinylpyrrolidone), poly(vinyl alcohol), poly(vinyl acetate), poly(ethylene-co-vinylacetate),
- Polyacetals,
- Polycarbonates,
- Polyethers, such as based on poly(oxyethylene) and poly(oxypropylene) units (Pluronic®),
- Aromatic polyesters, such as poly(ethylene terephthalate) (Dacron@), poly(propylene terephthalate) (Sorona®)
- Poly(ether ether ketone)s,
- Polysulfones,
- Silicone rubbers,
- Thermosets, such as epoxies,
- and Poly(ester imide)s

Representative examples of inorganics are listed below:
- Hydroxyapatite,
- Tricalcium phosphate,
- Silicates, such as Bioglass®, montmorillonite, and mica.

Typical examples of natural polymers include albumin, collagen, gelatin, hyaluronic acid, starch, alginate, pectin, cellulose and cellulose derivatives (such as methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextran, polysaccharides (such as sucrose acetate isobutyrate), and fibrinogen.

Additionally, materials as described herein also can be blended in various compositions as required.

The carriers―or depending on the form, the delivery vehicle--can be fashioned in a variety of forms with desired release characteristics and/or with specific desired properties. For example, the carriers may be fashioned to be in the form of a sheet, coating, slab, gel, capsule, microparticles, nanoparticles and combinations of these. For example, delivery vehicle 50 may contain a polymeric sheet loaded with the therapeutic agent as a carrier. Such a sheet may be formed by dissolving or dispersing both the polymer and therapeutic agent in a suitable solvent, pouring this solution in a suitable mould (e.g., glass petri dish), and removing the solvent by evaporation. The loaded sheet formed may be cut in such a way that it would fit the delivery vehicle.

Alternatively, delivery vehicle 50 may contain a gel as a carrier for the therapeutic agents. Such a gel may be prepared by dissolving the polymer in an organic solvent in which the therapeutic agent is either dissolved or dispersed. The gel-like liquid composition is placed into the delivery vehicle 50, and when the composition is implanted, the solvent diffuses into the body fluid. Subsequently a semi-solid implant is formed by the precipitation of the polymers in an aqueous environment. Such a gel releases the agent into the aneurysmal sac 18, where the delivery vehicle 50 provides a convenient mechanism to maintain the gel adjacent the aneurysmal sac 18.

If a gel is utilized, the agents described herein are intermixed with the gel, and the gel is preferably loaded into delivery vehicle 50 that provides a supporting structure to maintain the gel in a position adjacent to the aneurysmal sac 18. Within time, the therapeutic agent diffuses out of the gel and passes through the delivery vehicle and into contact with the aneurysmal sac 18. The gel may be prepared with a plurality of agents, or individual gel preparations may be prepared with individual agents and portions of the different gels intermixed. Alternatively, a gel or a combination of gels and other encapsulating members may be used in combination so that different agents are delivered at varying concentrations and over varying time courses in a manner that optimizes the therapeutic effect. Thus, multiple therapeutic agents, and customized relative percentages of agents and mixes of agents, may be provided for each patient.

After the delivery vehicle 50 is filled with the carrier―either the microspheres, a slab or a gel (described *infra*) or other form of carrier--end 58 is closed by sewing or other means and is attached to the stent graft 22, preferably at ring 48.

The position of the delivery vehicle 50 adjacent the aneurysmal sac 18, isolated by stent graft 22 from the blood passing through the aorta 10, establishes the delivery vehicle 50 in a relatively sealed environment such that blood and other fluids that may be in this region have a limited likelihood of being transmitted or passed from this region. Thus, the therapeutic agent will be released into the blood or other fluid in this relatively isolated region such that a maximum concentration of the agent likely will be reached in this region alone--the therapeutic agents are not be delivered to the rest of the body. As a result, substantial quantities of the therapeutic agent remain in place for a longer period of time, increasing the efficacy of the delivery of the agent. Figure 2B shows delivery vehicle 50 placed in a proximal position in the aorta, and illustrates a less advanced aneurysm. By placing delivery vehicle 50 at the proximal end of the stent graft 22, delivery vehicle 50 is positioned such that it is held in close contact with the aorta wall at that location, and thus at least a portion of the therapeutic agent will be released directly from the delivery vehicle and into contact with the aorta wall without having to pass first through the blood or other fluid in the aneurysmal sac 18. Therefore the therapeutic agent can be delivered directly, without intervening diffusion or release into the blood or other fluid, increasing its efficacy in treating the aneurysmal site.

Alternatively, multiple pouches may be used, with each pouch being attached by at least one end to the stent graft, and arranged such that the spacing between adjacent pouches extending about the circumference of the stent graft is, preferably, relatively equal. In one embodiment, at least four such delivery vehicles are equally spaced about the circumference of the stent graft when placed across the aneurysmal aorta. Alternatively, multiple delivery vehicles can be located both about the circumference of the stent graft, as well as longitudinally along stent graft.

To position the stent graft 22 in the aorta 10 and thereby provide a conduit to exclude blood flow to the aneurysmal sac 18, each of the trunk portion 38, the leg 40 and the leg 42 are delivered to the aneurysmal site 18 by endovascular means. In the embodiment of the stent graft 22 described herein, the stent graft 22 is a two piece stent graft, as the trunk 38 and leg 40 are formed as one continuous piece, or are connected together before deployment into the body.

To enable endovascular deployment of the stent graft, each of the unitary trunk 38 and leg 40, and the separate leg 42, are disposed in catheters and the catheters are inserted into and guided up an artery in the leg to the ascending aorta where the aneurysmal sac is to be bypassed. To enable placement of the two separate portions of the stent graft into the catheters, they are first flattened, and then rolled into a cylindrical body which can be inserted into the open end of the catheter. The catheter includes, at least, a sheath into which each of the stent graft portions are received, and a push rod or stopper, which bears against the inner end of the stent graft portion received in the sheath. The sheath is retractable with respect to the push rod, such that the portion of the stent graft 22, either the unitary trunk 38 and leg 40 or the second leg 42, remains stationary as the sheath is retracted, to enable the stent graft 22 to deploy in aorta 10.

To position the catheter for deployment of the stent graft 22, a guide wire is first inserted through a leg incision, and then directed along the artery until the end inserted into the aorta 10 extends beyond the deployment location for the stent graft 22. Then the catheter with the unitary trunk 38 and leg 40 portion therein is guided along the guide wire to the point where the end thereof is located upstream of the aneurysmal sac 18, and the sheath of the catheter is withdrawn as the push rod remains stationary. Thus, the trunk 38 and leg are deployed, such that the upper end of the trunk 38 is located above the aneurysmal sac 18, and the leg is extended down the left ilial artery 30. The trunk includes a cutout into which the right leg 42 of the stent graft, which will be guided from a second incision in the right leg of the patient, will be likewise guided. The inner end of the right leg 42 will be disposed in the opening in the trunk 38, and the second end therof will extend down and seal against the right iliac artery wall.

Therapeutic agents according to the present invention are those that are known in the art that are useful in treating aneurysms or that reduce the likelihood of progression of aneurysms. Such agents include, for example, matrix metalloproteinase (MMP) inhibitors, antibiotics, cyclooxygenase-2 (COX-2) inhibitors, angiotensin-converting enzyme (ACE) inhibitors, glucocorticoids, beta blockers, nitric acid synthase (NOS) inhibitors, antioxidants and cellular adhesion molecules (CAMs). The therapeutic agent or agents are released over time in the aneurysmal location, reducing the likelihood of further dilation and increasing the likelihood of successful repair of the aneurysm. It is contemplated that the therapeutic agent or agents may be supplied to the aneurysmal site with an excluding device such as a stent graft. Since the therapeutic agents are delivered locally, the dosage necessary for and side effects associated with oral or intravascular delivery are reduced substantially.

The therapeutic agents used in certain embodiments are those known to inhibit or block the production of proteins and enzymes known to be found in aneurysmal blood vessel locations, but which are not found--or are found at much lower concentrations--in healthy blood vessels. Such proteins and enzymes are at least in part responsible for, or are initiators or catalysts of biochemical compounds responsible for the loss of elasticity of or inflammation of blood vessels. Such loss of elasticity or inflammation contributes to aneurysm formation and progression. These therapeutic agents, when localized at the aneurysmal location, reduce the production of and/or occurrence of elastin-attacking proteins at the aneurysmal site. The therapeutic agents according to the present invention work through various mechanisms; for example, some work by altering biochemical pathways that produce compounds that cause inflammation, others work by inhibiting elastases, gelatinases or other proteinases or by preventing the adhesion of such molecules to the vascular cell wall.

For example, metalloproteinase 9 (MMP-9) is a protein that attacks elastin and causes breakdown of the medial tissue of the vasculature. Cyclooxygenase-2 or "COX-2" is known to burn a fat in the body known as arachidonic acid or AA, a naturally occurring omega-6 fatty acid found in nearly all cell membranes in humans. Prostaglandin E2 (PGE2) is synthesized from the catalyzation of COX-2 and arachidonic acid and, when PGE2 is taken up by macrophages, it results in MMP-9 formation. Thus, if any of COX-2, PGE2 or AA is suppressed, then MMP-9 formation will be suppressed. Therefore, one aspect according to the present invention provides MMP and COX-2 inhibitors at the aneurysmal site.

The key requirement for a potent inhibitor of MMPs is a functional group capable of chelating the active site zinc ion strongly, and embodiments according to the present invention use one or more of the major classes of MMP inhibitor compounds: hydroxamic acids, carboxylic acids, thiols, phosphinic acids, and tetracyclines. Typical MMP inhibitors include N-biphenyl sulfonyl-phenylalanine hydroxamic acid; amines, amino acid derivatives and low molecular weight peptides containing an amide-bound oxal hydroxamic acid moiety; benzodiazepine; acyclic succinic acid-based compounds; oleic acid; cerivastatin; thiol compound MAG-283; tetracycline derivatives, such as tetracycline, doxycycline (Periostat®), and 6-deoxy-6-demethyl-4-dedimethylamino-tetracycline (COL-3). Other suitable inhibitors include chelating agents, such as EDTA.

COX-2 inhibitors include Celecoxib, Rofecoxib, Valdecoxib, Etoricoxib, and Parecoxib, all of which are available in pharmacological preparations. Additionally, COX-2 inhibition has been demonstrated from herbs such as green tea, ginger, turmeric, chamomile, Chinese gold-thread, barberry, baikal skullcap, Japanese knotweed, rosemary, hops, feverfew, and oregano; and from other agents such as piroxican, mefenamic acid, meloxican, nimesulide, diclofenac, MF-tricyclide, raldecoxide, nambumetone, naproxen, herbimycin-A, and diaryl hydroxyfuranones. It is specifically contemplated in embodiments according to the present invention that such additional COX-2 inhibiting materials may be formulated for use in an aneurysmal location.

In addition to inhibiting COX-2 formation, the generation of elastin-attacking proteins may be limited by interfering with the oxidation reaction between COX-2 and AA by reducing the capability of AA to oxidize. It is known that certain NSAIDs provide this function. For example, ketoralac tromethamine (Toradol) inhibits synthesis of progstaglandins including PGE2. In addition, other currently available NSAIDs, including indomethacin, ketorolac, ibuprofen and aspirin, among others, reduce inflammation at the aneurysmal site, limiting the ability of elastin attacking proteins such as MMP-9 to enter into the cellular matrix of the blood vessel and degrade elastin. Additionally, steroidal based anti-inflammatories, such as methylprednisolone, dexamethasone or sulfasalazine may be provided to reduce the inflammation at the aneurysmal site. Other suitable anti-inflammatory agents include cyclosporine A and azathioprine. Another type of suitable therapeutic agents may be anti-oxidants, such as curcumin, vitamins, and vitamin constituents, such as α-tocopherol and β-carotene.

Despite the presence of inhibitors of COX-2 or of the oxidation reaction between COX-2 and AA; and/or despite the presence of an anti-inflammatory to reduce irritation and swelling of the blood vessel wall, MMP-9 may still be present in the blood vessel. Therefore, another class of therapeutic agents useful in embodiments according to the present invention is the class that limits the ability of elastin-attacking proteins to adhere to the blood vessel wall, such as anti-adhesion molecules. Anti-adhesion molecules, such as anti-CD18 monoclonal antibody, limit the capability of leukocytes that may have taken up MMP-9 to attach to the blood vessel wall, thereby preventing MMP-9 from having the opportunity to enter into the blood vessel cellular matrix and attack the elastin.

Other therapeutic agents useful in embodiments according to the present invention are angiotensin-converting enzyme (ACE) inhibitors that suppress the development of elastase-induced vessel damage. Such ACE inhibitors known in the art are captopril, enalapril, losartan and lisinopril and the active forms of several ACE inhibitor prodrugs on the market. Other agents such as the NOS inhibitor aminoguanidine are also useful in embodiments according to the present invention.

Another class of therapeutic agent that finds utility in embodiments according to the present invention for inhibiting the progression or inducing the regression of a pre-existing aneurysm is beta blockers or beta adrenergic blocking agents. Beta blockers that may be used in the compounds and methods of the present invention include acebutolol, atenolol, betaxolol, bisoprolol, carteolol, carvedilol, esmolol, labetolol, metoprolol, nadolol, penbutolol, pindolol, propranolol, and timolol, as well as other beta blockers known in the art.

While the foregoing is directed to embodiments according to the present invention, other and further embodiments of the invention may be devised without departing from the basic scope thereof.

All references cited herein are to aid in the understanding of the invention, and are incorporated in their entireties for all purposes.

## Claims

1. A device for the treatment of aneurysmal tissue, comprising:
at least one delivery vehicle locatable adjacent to an aneurysmal site; and
at least one time release carrier provided therewith, where the time release carrier is capable of dosing over time at least one therapeutic agent selected from cyclooxygenase-2 (COX-2) inhibitors, angiotensin-converting enzyme (ACE) inhibitors, glucocorticoids, beta blockers, nitric acid synthase (NOS) inhibitors, antioxidants, cellular adhesion molecules, or non-steroidal anti-inflammatory drugs (NSAIDs).

2. The device of claim 1, wherein the delivery vehicle is attached to a stent graft.

3. The device of claim 2, wherein delivery vehicle is a pouch.

4. The device of claim 2, wherein the time release carrier and at least one therapeutic agent are formulated as a sheet, a coating, a slab, a gel, capsules, microspheres, nanospheres and/or combinations thereof.

5. The device of claim 3, wherein the time release carrier and at least one therapeutic agent are formulated as a sheet, a coating, a slab, a gel, capsules, microspheres, nanospheres or combinations thereof and are placed in the pouch.

6. The treatment device of any of claims 1 to 5, wherein the time-release carrier, when implanted, releases the therapeutic agent into the aneurysmal site.

7. The device of any of claims 1 to 6, wherein the time release carrier consists of a synthetic biodegradable polymer, a synthetic biostable polymer, a natural polymer, an inorganic material or combinations thereof.

8. The device of claim 7, wherein the biodegradable polymer is an aliphatic polyester, a poly(ortho ester), a poly(ester amide), a poly(ester urethane), a poly(ester anhydride), a poly(ester carbonate), a polyphosphazene, a polyarylate, a poly(ether ester), and/or combinations thereof.

9. The device of claim 7, wherein the biostable polymer is a polyolefin, a polyurethane, a fluorinated polyolefin, a chlorinated polyolefin, a polyamide, an acrylate polymer, an acrylamide polymer, a vinyl polymer, a polyacetal, a polycarbonate, a polyether, an aromatic polyester, a poly(ether ether ketone), a polysulfone, a silicone rubber, a thermoset, or a poly(ester imide) and/or combinations thereof.

10. The device of claim 7, wherein the natural polymer is albumin, collagen, gelatin, hyaluronic acid, starch, alginate, pectin, cellulose and cellulose derivatives, casein, dextran, polysaccharides, or fibrinogen and/or combinations thereof.

11. The device of any of claims 3 to 5, wherein the pouch is comprised of a polymeric material.

12. The device of claim 11, wherein the polymeric material is a polyolefin, a polyurethane, a silicone, a polyester or a fluorinated polyolefin.

13. The device of any of claims 1 to 12, wherein the time release carrier comprises a combination of therapeutic agents.

14. The device of claim 2, wherein the time release carrier is attached to the stent graft between the stent graft and the aneurysmal site.

15. The device of claim 3, wherein the pouch is attached to the stent graft between the stent graft and the aneurysmal site.

16. A device for the treatment of aneurysmal tissue, comprising: a delivery vehicle locatable adjacent to an aneurysmal site, wherein the delivery vehicle has a form of a sheet, slab, gel and/or combinations thereof, and comprises at least one time release carrier and at least one therapeutic agent selected from cyclooxygenase-2 (COX-2) inhibitors, angiotensin-converting enzyme (ACE) inhibitors, glucocorticoids, beta blockers, nitric acid synthase (NOS) inhibitors, antioxidants, cellular adhesion molecules, or non-steroidal anti-inflammatory drugs (NSAIDs).

17. The device of claim 16, wherein the delivery vehicle is attached to a stent graft.

18. The treatment device of claim 16 or 17, wherein the delivery vehicle, when implanted, releases the therapeutic agent into the aneurysmal site.

19. The device of any of claims 16 to 18, wherein the time release carrier consists of a synthetic biodegradable polymer, a synthetic biostable polymer, a natural polymer, an inorganic material or combinations thereof.

20. The device of claim 19, wherein the biodegradable polymer is an aliphatic polyester, a poly(ortho ester), a poly(ester amide), a poly(ester urethane), a poly(ester anhydride), a poly(ester carbonate), a polyphosphazene, a polyarylate, a poly(ether ester), and/or combinations thereof.

21. The device of claim 19, wherein the biostable polymer is a polyolefin, a polyurethane, a fluorinated polyolefin, a chlorinated polyolefin, a polyamide, an acrylate polymer, an acrylamide polymer, a vinyl polymer, a polyacetal, a polycarbonate, a polyether, an aromatic polyester, a poly(ether ether ketone), a polysulfone, a silicone rubber, a thermoset, or a poly(ester imide) and/or combinations thereof.

22. The device of claim 19, wherein the natural polymer is albumin, collagen, gelatin, hyaluronic acid, starch, alginate, pectin, cellulose and cellulose derivatives, casein, dextran, polysaccharides, or fibrinogen and/or combinations thereof.

23. The device of any of claims 16 to 22, wherein the at least one therapeutic agent comprises a combination of therapeutic agents.

24. The device of claim 17, wherein the delivery vehicle is attached to the stent graft between the stent graft and the aneurysmal site.
